# EUROPEAN PATENT SPECIFICATION

(11) **EP 0 577 205 B1**
(45) Date of publication and mention of the grant of the patent: **08.10.1997**
(21) Application number: 93201843.5
(22) Date of filing: 25.06.1993
(51) Int. Cl.: C07C 67/38, C07C 69/533

(54) **Carbonylation of conjugated dienes**
Carbonylierung von conjugierten Dienen
Carbonylation de diènes conjugués

(30) Priority: 29.06.1992 EP 92201941
(43) Date of publication of application: 05.01.1994
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL); Jager, Willem Wabe, NL-1031 CM Amsterdam (NL)

(56) References cited:
- EP-A- 0 271 145
- EP-A- 0 273 489
- EP-A- 0 284 170

## Description

This invention relates to a process for the preparation of an alkenoic acid derivative by reaction of an aliphatic conjugated diene with carbon monoxide and a hydroxyl group-containing compound in the liquid state in the presence of a catalyst system comprising a palladium compound, a multidentate phosphine ligand, and optionally a protonic acid.

Products obtainable by the present process, such as alkenoic esters, acids and anhydrides, constitute versatile intermediates for various chemical products, which thus become accessible using cheap base feedstock, such as butadiene. Alkyl 3-pentenoates, for example, are intermediates to dialkyl adipates and 6-oxohexanoates in an efficient manufacture route to polyamide-6,6 and polyamide-6, respectively.

US-A-4172087 discloses a process for the carbonylation of conjugated dienes in the presence of an alcohol and using a catalyst system comprising a halide-free palladium salt, a multidentate phosphine ligand and tertiary nitrogen base, for instance pyridine, quinoline or isoquinoline. The tertiary nitrogen base is used in large excess relative to the further catalyst components, and essentially serves as the solvent for the reaction. In the absence of added N-heterocyclic solvent, considerable precipitation of palladium-containing insoluble species and much lower product yields are reported. This known process involves two concurrent reactions, viz. dimerisation and carbonylation, and invariably mixed reaction products, for instance isopropyl pentenoate and isopropyl nonadienoate, were obtained.

A subsequent improvement in terms of selectivity of the conjugated diene carbonylation was disclosed by EP-A-273489, according to which the reaction is to be carried out in the presence of a specific substantially organic nitrogen-containing base-free catalyst system comprising a palladium compound in conjunction with at least one multidentate organic phosphorus ligand. According to a preferred embodiment, a catalytic quantity of a protonic acid with a pKa value > 3 may be added to increase the yield of, for example, pentenoates, in the case of conversion of butadiene, emphasising the desirability of avoiding the basic reaction medium used according to US-A-4172087. The process of EP-A-273489 affords the carbonylation of butadiene to proceed with a selectivity to alkyl pentenoates of about 90 % or higher.

Whereas the known processes provide an interesting route to alkenoic acid derivatives from cheap feedstock, their reaction rates in relation to the amount of precious palladium catalyst component applied leaves room for further improvement for providing an industrially viable process, which can compete with commercial technology, in particular for the production of polyamides. The present invention aims at achieving such improvement.

It has now been found that the above object is attained by conducting the reaction in a reaction medium of controlled polarity.
Surprisingly, it appears that careful control of the polarity of the medium during the course of the carbonylation reaction allows for higher reaction rates being achieved than were possible hitherto, whilst maintaining excellent selectivities, even when an organic nitrogen-containing base is present. According to the invention, high polarities of the reaction medium as retarding the reaction, are to be avoided. Too low a polarity of the reaction medium may not be suitable either, in view of reduced solubility of the catalyst metal component.

Whereas the present process may be carried out whilst continuously monitoring the polarity of the liquid reaction medium, the present invention also provides a convenient criterion for assessment of the polarity required for the reaction medium. According to the present process, the calculated dielectric constant ε_{calc} of the reaction medium is below a value of 8, preferably at a value in the range of from 4.5 to 7.5, most preferably in the range of from 5 to 6.5 during the course of the reaction. To this end, ε_{calc} is calculated as the volume average of the dielectric constants at 25 °C of the pure liquid substrates and solvents present in the reaction medium, which data are available from literature. Though in this approach minor influences from dissolved amounts of carbon monoxide and catalyst components are neglected, for practical purposes the criterion has appeared to be sufficiently accurate for distinguishing the polarity condition required for improved reaction rates of the carbonylation reaction. The prior processes all were conducted in a reaction medium of ε_{calc} above 8.

The liquids to be taken into account for calculating ε_{calc} usually will be the aliphatic conjugated diene, the hydroxyl group-containing compound and the solvent or solvents, if any. It is conceivable, that alkenoic acid derivative product formed during the course of the reaction, may contribute to the average dielectric constant of the liquid reaction mixture. However, the dielectric constants of these products incidentally fall in the range of from about 5 to about 6.5, which is preferred for the liquid reaction mixture and therefore will have a minor or negligible contribution to the average dielectric constant, when the process is carried out in accordance with the invention.

In the present context, the dielectric constant for a given liquid is used in its normal meaning of representing the ratio of the capacity of a condenser with that substance as dielectric to the capacity of the same condenser with a vacuum for dielectric. Values for the dielectric constants of common organic liquids may be found in general reference books, such as the Handbook of Chemistry and Physics, Edited by CRC, or more specialised publications, and are usually quoted for a temperature of 25 °C, or can be readily converted to that temperature using the conversion factors quoted. If no literature data for a particular compound are vailable, the dielectric constant may be readily measured using established physico-chemical methods. For example, for ethanol ε = 24.3, for diphenyl ether ε = 3.7 and for 1,3-butadiene ε = 1.9 (For butadiene, by extrapolation of the data in J.Chem. Thermodynamics, **1986**, 18, 221-234). On this basis, a volume averaged dielectric contant ε_{calc} of 8.4 is calculated for a mixture of 15 ml ethanol, 40 ml diphenyl ether and 8 ml butadiene (15/63 x 24.3 + 40/63 x 3.7 + 8/63 x 1.9 = 8.4) as used in Example 1 of EP-A-273489. Similarly, a reaction medium of 40 ml pyridine (ε = 12.3), 20 ml isopropanol (ε = 18.3), and 32 ml 1,3-butadiene, as used in Example 12 of US-A-4172087, has an ε_{calc} of 10.0. If containing quinoline or isoquinoline, as in Examples 1 or 3 of US-A-4172087, the reaction medium has an ε_{calc} of 8.6 or 9.3, respectively.

Substrates, solvents and catalyst components, which can be used in the present process are essentially the same as used in the prior processes. However, by judicious combination of the same and/or selection of the concentrations following the teaching of the present invention, the polarity of the reaction medium can be controlled such, that the desired increase of reaction rate is achieved.

Suitable aliphatic conjugated dienes include alkadienes, preferably having from 4 to 8 carbon atoms, such as 1,3-butadiene (ε = 1.9), 1,3-pentadiene (ε = 2.32), 1,3-hexadiene, cis,cis-2,4-hexadiene (ε = 2.16), trans,trans-2,4-hexadiene (ε = 2.12), 1,3-cyclohexadiene or 2,4-heptadiene, which may carry substituents not interfering with carbonylation reaction, for example alkyl substituents, such as in 2-methyl-1,3-butadiene (ε = 2.10).

Representative hydroxyl group-containing compounds include alcohols, carboxylic acids and water, of which the alcohols yielding alkenoic esters in the carbonylation reaction are preferred. The alcohols may be aliphatic or aromatic, such as phenol. The use of water yields alkenoic acids, and the use of carboxylic acids alkenoic anhydrides. Typical examples include methanol (ε = 32.6), ethanol (ε = 24.3), isopropanol (ε = 18.3), n-butanol (ε= 17.1), sec-butanol (ε = 15.8), isobutyl alcohol (ε = 17.7) and tert.-butanol (ε = 11.5). The alcohol may be polyfunctional, such as ethylene glycol (ε = 37.7), 1,3-propanediol (ε = 35.0) or glycerol (ε = 42.5).

The preferred substrates for the present process are 1,3-butadiene in conjunction with an alkanol having from 1 to 4 carbon atoms, which by the reaction with carbon monoxide yield alkyl esters of pentenoic acid, in particular of the 3-pentenoic acid isomer.

It will be appreciated that the alcohol reactants significantly contribute to the average dielectric constant, in particular methanol and ethanol. For controlling the polarity of the reaction medium it is therefore preferred according to a first embodiment of the invention, that in the carbonylation process the hydroxyl group-containing compound is an alkanol having an dielectric constant at 25 °C of below 20, more preferably the alkanol is isopropanol or tert-butanol.

According to an alternative embodiment of the invention, the polarity of the reaction medium can also be controlled when the hydroxyl group-containing compound is an alkanol having an dielectric constant at 25 °C of above 20 by assuring that its concentration does not exceed 20 %vol of the liquid reaction medium during the course of the reaction. This may be achieved by dilution of the reaction mixture with relatively large amounts of low polar solvent with concurrent reduction of the concentration of the other substrate. This may render the process unduly cumbersome in view of excessively large liquid streams including any recycling. More preferably, the polarity is controlled by starting the reaction at low concentration of specifically the alkanol, which may be substoichiometric, the converted amounts of alkanol being repleted by continuous or intermittent further addition of alkanol during the course of the reaction. Accordingly, the cheaper alcohols such as ethanol can also be used in the present process, so that particularly methyl and ethyl esters may be prepared, if so desired.

If it is desired to use an alkanol having an dielectric constant at 25 °C of above 30, such as methanol, most preferably it is continuously or intermittently added during the course of the reaction, such that its concentration does not exceed 12 %vol of the liquid reaction medium.

The catalyst system to be used in the present process comprises a palladium compound which may be derived from any source of palladium, which will dissolve in the reaction medium to a sufficient extent for forming the catalytically active species with the further catalyst components. Suitable sources of palladium include salts, such as palladium acetate, palladium acetylacetonate, palladium sulphate and palladium nitrate, and coordination complexes, such as tetrakis triphenylphosphine palladium. Preferably the source of palladium is free of halide. Palladium may be used in a heterogenised form, for example loaded on an ion exchange resin containing sulphonic acid groups.

The multidentate phosphine ligand contains at least two phosphine P atoms being interconnected by a bridging group allowing for bidentate coordination of the ligand to the palladium metal atom. Very suitable ligands are those of formula R₁R₂>P-R-P<R₃R₄, wherein R₁, R₂, R₃ and R₄ represent hydrocarbyl groups, for example aryl or (cyclo)alkyl groups, optionally substituted with one or more substituents and R represents a divalent organic bridging group with at least two carbon atoms forming the bridge. Preferably R represents an alkylene group having three or four carbon atoms, most preferably four carbon atoms. Examples of particularly suitable multidentate phosphine ligands are:
1,3-di(diphenylphosphino)propane, 1,4-di(diphenylphosphino)butane and 1,4-di(di-n-butylphosphino)butane. The molar ratio of multidentate phosphine ligand to palladium gram atoms generally is in the range of from 1 to 10. Other phosphines, in particular monophosphines, may additionally be present in the catalyst system.

The catalyst system may further, as an optional component, comprise a weak protonic acid, for example having a pKa > 3. The protonic acid preferably is a stericly hindered carboxylic acid, such as 2,4,6-trimethylbenzoic acid, 2,6-dichlorobenzoic acid or 9-anthroic acid. In general, the amount of protonic acid used is in the range of from 2 to 50 equivalents of acid per gram atom of palladium. The protonic acid may be neutralised in part or entirely by the addition of a base, for example an organic tertiary nitrogen base, provided the latter is present in a limited concentration of, for example, below 5 %vol, so that the polarity of the reaction medium remains within the controlled values.

A separate solvent is not essential for the process according to the present invention, when an excess of one of the reactants or the product will form a suitable liquid phase. From the above it will be appreciated that the use as carrier of an excess of the alcohol substrate usually will not be effective. In some cases, it may be desirable to use a separate solvent. Any inert solvent can, in principle, be used for this purpose. As solvents will generally be used in fairly large amounts, it is preferred that a solvent of limited polarity is used, such as an ether. Typical suitable solvents include aromatic hydrocarbons such as benzene (ε = 2.27), toluene (ε = 2.4) and xylenes; esters such ethyl acetate (ε = 6.02), methyl propionate (ε = 5.5) and pentenoates; and ethers such as anisole, diglyme (2,5,8-trioxanonane), diphenyl ether (ε = 3.7) and diisopropyl ether or mixtures thereof. The use of more polar aromatic N-heterocycles as solvent in amounts of, say, higher than 10 %vol should be avoided.

Reaction conditions of temperature and pressure are the same as for previously described processes. Temperatures of from 20 to 200 °C, more particular of from 50 to 150 °C are most suitable. Pressures of from 5 to 100, more particular of from 25 to 65 bar are typical. Carbon monoxide grade, reaction equipment and product purification are not critical, and well within the skills of the relevant technician.

The invention will be further illustrated in detail by the following non-limiting examples.

### Example 1

A 300 ml magnetically stirred stainless steel autoclave was charged with 30 ml tert-butanol (0.32 mol), 40 ml diphenyl ether, 0.5 mmol palladium acetate, 3 mmol 1,4-di(diphenylphosphino)butane, and 10 mmol 2,4,6-trimethylbenzoic acid. The autoclave was flushed and evacuated, whereupon 20 ml of 1,3-butadiene was added and carbon monoxide was introduced to an initial carbon monoxide pressure of 30 bar. For this reaction mixture, ε_{calc} is 5.9. The autoclave was heated to 140 °C and the rate of pressure decrease due to carbon monoxide consumption was recorded. An initial reaction rate over the first hour of reaction of 550 mol of converted butadiene per gram atom of palladium per hour was observed. After a total reaction time of 5 hours, the autoclave was cooled and its contents analysed by means of gas liquid chromatography (GLC). 1,3-Butadiene was found to be converted to t-butyl pentenoates with a selectivity of 95 %, of which about 90 % was constituted by the 3-isomers.

### Comparative Example

Example 4 of EP-A-273489 was repeated by charging an autoclave with 15 ml ethanol, 40 ml diphenyl ether, 8 ml 1,3-butadiene, 1 mmol palladium acetate, 4 mmol 1,4-di(diphenylphosphino) butane and 7.5 mmol 2,4,6-trimethylbenzoic acid. The ε_{calc} for this reaction mixture is 8.4. The autoclave was heated at a temperature of 150 °C and an initial reaction rate of 60 mol converted butadiene per gram atom palladium per hour was recorded.

### Examples 2-6

Example 1 was repeated except for using the alcohols and their amounts in ml and mole as indicated in the below Table. As catalyst a system was used comprising A: 0.5 mmol palladium acetate, 1.5 mmol 1,4-di(diphenylphosphino)butane, and 5 mmol 2,4,6-trimethylbenzoic acid; B: 0.5 mmol palladium acetate, 3 mmol 1,4-di(diphenylphosphino)butane, and 10 mmol 2,4,6-trimethylbenzoic acid, or C: 0.5 mmol palladium acetate, 3 mmol 1,4-di(diphenylphosphino)butane, and 10 mmol 2,4,6-trimethylbenzoic acid, as indicated. The Table further mentions ε_{calc} and the observed initial reaction rate for each Example. Selectivities to the respective alkyl pentenoates and 3-isomer contents were essentially the same as in Example 1. It is seen that high reaction rates are obtained when ε_{calc} is in the range according to the invention. Examples 7 and 8 are outside the scope of the invention, but show that some increase of reaction rate relative to Comparative Example can be obtained by increasing the concentration of the butadiene substrate. They also show that the much larger improvement achieved by the invention cannot satisfactorily be explained by an increase of butadiene activity in the reaction.

**TABLE**

| Example No. | Alcohol | amount | | catalyst type | ε_{calc} | rate mole/gat/h |
|---|---|---|---|---|---|---|
| | | ml | mole | | | |
| 2 | t-butanol | 30 | 0.32 | B | 5.9 | 470 |
| 3 | methanol | 5 | 0.12 | C | 5.4 | 390 |
| 4 | s-butanol | 30 | 0.33 | A | 7.3 | 275 |
| 5 | t-butanol | 15 | 0.16 | C | 4.8 | 240 |
| 6 | n-butanol | 30 | 0.33 | B | 7.8 | 200 |
| 7 | ethanol | 20 | 0.34 | A | 8.4 | 190 |
| 8 | methanol | 20 | 0.49 | B | 10.4 | 100 |

### Example 9

Example 1 was essentially repeated except for charging 20 ml tert-butanol and 10 ml methanol instead of 30 ml of tert-butanol. For this reaction mixture, ε_{calc} is 8.2. An initial reaction rate of 160 mole/gram atom/hour, a selectivity to methyl pentenoates of 95 %, and a 3-isomer proportion of 90 % were observed. This Example is for illustrative purposes and not according to the invention.

### Example 10

Example 9 was repeated except for using a mixture 25 ml tert-butanol and 5 ml methanol as the hydroxyl group-containing compound. For this reaction mixture, ε_{calc} is 7.1. An initial reaction rate of 315 mole/gram atom/hour was observed, the initial selectivity and 3-isomer content being the same as in Example 9.

It is seen from Examples 9 and 10, that the methyl rather than the tert-butyl esters are preferentially formed. Therefore, it is concluded that any imaginable higher reactivity of higher alcohols such as tert-butanol or isopropanol relative to lower alcohols such as ethanol or methanol would not provide a satisfactory explanation for the higher reaction rates observed in Examples 1 to 6 in accordance with the invention.

### Example 11

A 300 ml magnetically stirred stainless steel autoclave was charged with 30 ml tert-butanol (0.32 mol), 40 ml diphenyl ether, 0.5 mmol palladium acetate, 3 mmol 1,4-di(diphenylphosphino)butane, and 15 mmol 2,4,6-trimethylbenzoic acid. Furthermore, 1.2 ml of 3,4-lutidine (10 mmol) were added to the reaction mixture. The autoclave was flushed and evacuated, whereupon 20 ml of 1,3-butadiene was added and carbon monoxide was introduced to an initial carbon monoxide pressure of 30 bar. The autoclave was heated to 140 °C and the rate of pressure decrease due to carbon monoxide consumption was recorded. An initial reaction rate over the first hour of reaction of 550 mol of converted butadiene per gram atom of palladium per hour was observed. After a total reaction time of 1.5 hours, the autoclave was cooled and its contents analysed by means of gas liquid chromatography (GLC). 1,3-Butadiene was found to be converted to t-butyl pentenoates with a selectivity of 95 %, of which 92 % was constituted by the 3-isomers.

It is seen that the addition of small amounts of N-heterocyclic bases does not affect the selectivity of the present process. In fact, a slight improvement of the reaction rate is observed. Since these small amounts of N-heterocyclic base are about equimolar to the acid component of the catalyst system, this embodiment of the invention allows for conducting the carbonylation process under non-acidic conditions.

### Example 12

Example 11 was essentially repeated except for adding 2.5 ml of 2,6-di-tert-butylpyridine (10 mmol) instead of 1.2 ml of 3,4-lutidine. An initial reaction rate of 550 mmol/gram atom/hour was observed.

### Example 13

A 250 ml magnetically stirred stainless steel autoclave was charged with 30 ml tert-butanol (0.32 mol), 40 ml diphenyl ether, 0.5 mmol palladium acetate, 3 mmol 1,4-di(diphenylphosphino)butane, and 15 mmol 2,4,6-trimethylbenzoic acid. Furthermore, 0.6 ml of 3,4-lutidine (5 mmol) were added to the reaction mixture. The autoclave was flushed and evacuated, whereupon 20 ml of a C⁴-mixture comprising 11 %mol of butanes, 47 %mol of butenes and 42 %mol of butadienes was added and carbon monoxide was introduced to an initial carbon monoxide pressure of 30 bar. The autoclave was heated to 140 °C for 5 hours, whereupon the autoclave was cooled and the pressure released. The gaseous effluent of the autoclave was analysed and found to contain 11 %mol of butadienes on the basis of total C⁴ content. Its liquid content was analysed by means of GLC. The carbonylation products were found to consist for 97 %mol of tert-butyl pentenoates with no pentanoates as conceiveable butene carbonylation products being detected.

It is seen that butadiene can selectively be carbonylated in the presence of butenes using the present process. This is a substantial advantage as C⁴ mixture feedstock is readily available from cracker operations as the so-called BBB stream.

## Claims

1. A process for the preparation of an alkenoic acid derivative by reaction of an aliphatic conjugated diene with carbon monoxide and a hydroxyl group-containing compound in the liquid state in the presence of a catalyst system comprising a palladium compound, a multidentate phosphine ligand, and optionally a protonic acid, wherein the reaction is conducted in a reaction medium of controlled polarity, having a calculated dielectric constant ε_{calc} (calculated as the volume average of the dielectric constants at 25 °C of the aliphatic conjugated diene, the hydroxyl group-containing compound and any further liquid) that is below a value of 8.

2. A process as claimed in claim 1, wherein the calculated dielectric constant of the reaction medium is maintained at a value of ε_{calc} in the range of from 4.5 to 7.5.

3. A process as claimed in any one or more of claims 1-2, wherein the hydroxyl group-containing compound is an alkanol having an dielectric constant ε at 25 °C of below 20.

4. A process as claimed in claim 3, wherein the alkanol having an dielectric constant ε at 25 °C of below 20, is tert-butanol.

5. A process as claimed in any one or more of claims 1-2, wherein the hydroxyl group-containing compound is an alkanol having an dielectric constant at 25 °C of above 20, of which the concentration is maintained below 20 %vol of the liquid reaction medium during the course of the reaction.

6. A process as claimed in claim 5, wherein the hydroxyl group-containing compound is an alkanol having a dielectric constant ε at 25 °C of above 30, of which the concentration is maintained below 12 %vol of the liquid reaction medium during the course of the reaction.

7. A process as claimed in claim 5 or 6, wherein further amounts of the alkanol are continuously or intermittently added during the course of the reaction at an rate substantially corresponding with the consumption of the alkanol.

8. A process as claimed in any one or more of claims 1-7, wherein use is made of a solvent having an dielectric constant ε at 25 °C of below 6.

9. A process as claimed in claim 8, wherein the solvent is an ether.

10. A process as claimed in any one or more claims 1-9, wherein an organic nitrogen-containing base is present at a concentration of below 5 %vol.

## Patentansprüche

1. Verfahren zur Herstellung eines Alkensäurederivats durch Umsetzung eines aliphatischen konjugierten Diens mit Kohlenmonoxid und einer hydroxylgruppenhaltigen Verbindung in flüssigem Zustand in Gegenwart eines Katalysatorsystems, enthaltend eine Palladiumverbindung, einen mehrzähnigen Phosphinliganden und gegebenenfalls eine Protonensäure, bei dem man die Umsetzung in einem Reaktionsmedium mit gesteuerter Polarität, das eine berechnete Dielektrizitätskonstante ε_{ber} (berechnet als das Volumenmittel der Dielektrizitätskonstanten des aliphatischen konjugierten Diens, der hydroxylgruppenhaltigen Verbindung und jeglicher weiterer Flüssigkeit bei 25°C) unter 8 aufweist, durchführt.

2. Verfahren nach Anspruch 1, bei dem man die berechnete Dielektrizitätskonstante des Reaktionsmediums bei einem ε_{ber}-Wert im Bereich von 4,5 bis 7,5 hält.

3. Verfahren nach einem oder mehreren der Ansprüche 1-2, bei dem man als hydroxylgruppenhaltige Verbindung ein Alkanol mit einer Dielektrizitätskonstante ε bei 25°C unter 20 einsetzt.

4. Verfahren nach Anspruch 3, bei dem man als Alkanol mit einer Dielektrizitätskonstante ε bei 25°C unter 20 tert.-Butanol einsetzt.

5. Verfahren nach einem oder mehreren der Ansprüche 1-2, bei dem man als hydroxylgruppenhaltige Verbindung ein Alkanol mit einer Dielektrizitätskonstante ε bei 25°C über 20 einsetzt und dessen Konzentration im Verlauf der Reaktion unter 20 Vol.-%, bezogen auf das flüssige Reaktionsmedium, hält.

6. Verfahren nach Anspruch 5, bei dem man als hydroxylgruppenhaltige Verbindung ein Alkanol mit einer Dielektrizitätskonstante ε bei 25°C über 30 einsetzt und dessen Konzentration im Verlauf der Reaktion unter 12 Vol.-%, bezogen auf das flüssige Reaktionsmedium, hält.

7. Verfahren nach Anspruch 5 oder 6, bei dem man im Verlauf der Reaktion weitere Mengen des Alkanols mit einer Geschwindigkeit, die im wesentlichen dem Verbrauch des Alkanols entspricht, kontinuierlich oder diskontinuierlich zusetzt.

8. Verfahren nach einem oder mehreren der Ansprüche 1-7, bei dem man ein Lösungsmittel mit einer Dielektrizitätskonstante ε bei 25°C unter 6 einsetzt.

9. Verfahren nach Anspruch 8, bei dem man als Lösungsmittel einen Ether einsetzt.

10. Verfahren nach einem oder mehreren der Ansprüche 1-9, bei dem man eine organische stickstoffhaltige Base in einer Konzentration unter 5 Vol.-% einsetzt.

## Revendications

1. Procédé de préparation d'un dérivé d'acide alcénoïque par la réaction d'un diène aliphatique conjugué avec le monoxyde de carbone et un composé contenant des radicaux hydroxyle, à l'état liquide et en présence d'un système catalytique comprenant un composé du palladium, un ligand du type phosphine multidentate et, éventuellement, un acide protonique, caractérisé en ce que l'on entreprend la réaction dans un milieu réactionnel de polarité réglée, possédant une constante diélectrique calculée ε_{calc} (calculée sous forme de la moyenne volumique des constantes diélectriques à 25°C du diène aliphatique conjugué, du composé contenant des radicaux hydroxyle et de tout autre liquide supplémentaire) qui a une valeur inférieure à 8.

2. Procédé suivant la revendication 1, caractérisé en ce que la constante diélectrique calculée du milieu réactionnel est maintenue à une valeur de ε_{calc} dans la plage de 4,5 à 7,5.

3. Procédé suivant l'une quelconque des revendications 1 et 2, caractérisé en ce que le composé contenant des radicaux hydroxyle est un alcanol possédant une constante diélectrique ε à 25°C, inférieure à 20.

4. Procédé suivant la revendication 3, caractérisé en ce que l'alcanol possédant une constante diélectrique ε à 25°C, inférieure à 20 est le tert-butanol.

5. Procédé suivant l'une quelconque des revendications 1 et 2, caractérisé en ce que le composé contenant des radicaux hydroxyle est un alcanol possédant une constante diélectrique à 25°C, supérieure à 20, dont on maintient la concentration en dessous de 20% en volume du milieu réactionnel liquide, au cours du déroulement de la réaction.

6. Procédé suivant la revendication 5, caractérisé en ce que le composé contenant des radicaux hydroxyle est un alcanol possédant une constante diélectrique ε à 25°C, supérieure à 30, dont on maintient la concentration en dessous de 12% en volume du milieu réactionnel liquide, au cours du déroulement de la réaction.

7. Procédé suivant la revendication 5 ou 6, caractérisé en ce que des quantités supplémentaires de l'alcanol sont ajoutées en continu ou par intermittences, au cours du déroulement de la réaction, à un débit correspondant sensiblement à la consommation de l'alcanol.

8. Procédé suivant une ou plusieurs des revendications 1 à 7, caractérisé en ce que l'on utilise un solvant possédant une constante diélectrique ε à 25°C, inférieure à 6.

9. Procédé suivant la revendication 8, caractérisé en ce que le solvant est un éther.

10. Procédé suivant une ou plusieurs des revendications 1 à 9, caractérisé en ce qu'une base contenant de l'azote organique est présente en une concentration inférieure à 5% en volume.
